# EUROPEAN PATENT APPLICATION

(11) **EP 2 716 359 A1**
(43) Date of publication of application: **09.04.2014**
(21) Application number: 12788876.6
(22) Date of filing: 23.05.2012
(51) Int. Cl.: B01J 20/02, C02F 1/28, C22B 3/18, C22B 11/00, C22B 58/00, C22B 59/00, C12N 1/20

(54) **COMPLEX AND USES THEREOF**

(30) Priority: 26.05.2011 JP 2011118190; 27.04.2012 JP 2012102888
(71) Applicant: Aisin Seiki Kabushiki Kaisha, Kariya-shi, Aichi 448-8650 (JP)
(72) Inventor: OTSUBO, Isao, Kariya-shi Aichi 448-8650 (JP); KONISHI, Yasuhiro, Sakai-shi Osaka 599-8531 (JP); SAITO, Norizo, Sakai-shi Osaka 599-8531 (JP)
(74) Representative: TBK
(86) International application number: PCT/JP2012/063172
(87) International publication number: WO 2012/161216

(57) **Abstract**

The present invention provides a complex of a metal reducing substance and a culture medium component, which is produced in the culturing process of reducing bacteria and the use thereof. The complex of the present invention is a complex produced in the culturing process of reducing bacteria and a compound of a metal reducing substance and a culture medium component. The complex is an adsorbent for quickly and efficiently adsorbing ionic substances present in a solution, and also functions as a reducing agent for reducing specific precious metal ions or platinum-group metal ions.

## Description

### TECHNICAL FIELD

The present invention relates to a complex of a metal reducing substance and a culture medium component, which is produced in the culturing process of reducing bacteria and the invention also relates to the use thereof.

### BACKGROUND ART

In recent years, in view of environmental pollution problems, environmental clarification materials for removing heavy metals and the like contained in wastewater and the like have attracted attention. The coagulation sedimentation method using, for example, iron salt as an environmental cleaning material is known. However, the coagulation sedimentation method has a problem that, depending on the valances of metals, cannot remove heavy metals enough.

To solve this problem, a reducing water quality clarification material containing at least any of iron ferrite and reducing iron hydroxide together with green rust has been developed (please refer to, for example, Patent Document 1). When this reducing water quality clarification material is used, heavy metals contained in wastewater can be effectively removed.

However, this reducing water quality clarification material exerts its metal recovering function only under neutral to alkaline conditions. Therefore, acid wastewater and the like cannot be directly processed and pre-treatments such as alkaline treatment are required. The alkaline treatment has a problem that processing equipment or chemicals is required therefor.

In addition, the reducing water quality clarification material needs to prevent its oxidation during the production process. For this purpose, the reducing water quality clarification material is subjected to deoxidation treatment in a closed vessel and repeated complicated pH treatments (alkaline treatment). Therefore, the production becomes complicated.

Further, in recent years, rare metals, precious metals, platinum-group metals, rare earth and the like are used for a semiconductor material, solder paste or electronic devices such as a liquid crystal display. Many of these metals exist as a mineral which intrinsically contains a small amount of the metal, or as a minor constituent which is contained in other minerals than the mineral intrinsically containing the corresponding metal. Therefore, a method of effectively recovering these metals is desired.

As a method of recovering precious metals and platinum-group metals such as gold and platinum, a method using a synthetic resin adsorbent such as an ion-exchange resin and a chelating resin is known. However, the synthetic resin adsorbents such as ion-exchanging resins are expensive and require processes such as regeneration thereof. Therefore, adsorbents which can be easily treated and can reduce precious metals to recover the same are desired.

On the other hand, the inventors have developed a method for reducing precious metal ions or platinum-group metal ions to recover precious metals or platinum-group metals by using iron-reducing bacteria (please refer to, for example, Patent Document 2). Further, the inventors have proposed a method of recovering metals where iron-reducing bacteria are used to recover indium, gallium or tin from metal containing materials which contain indium, gallium or tin (please refer to, for example, Patent Document 3). However, when iron-reducing bacteria are used, the methods are affected by culturing conditions and the like. Therefore, it is more preferable if there is a metal recovering material which has a preserving property and can be stably supplied.

### PRIOR ART DOCUMENT

### PATENT DOCUMENTS

Patent Document 1: JP 2006-289338 A
Patent Document 2: JP 2007-113116 A
Patent Document 3: JP 2011-26701 A

### DISCLOSURE OF THE INVENTION

### PROBLEM TO BE SOLVED BY THE INVENTION

The present invention has been made in light of the above problems and an object thereof is to provide a complex of a metal reducing substance and a culture medium component, which is produced in the culturing process of reducing bacteria and the present invention also relates to the use thereof.

### MEANS TO SOLVE THE PROBLEM

The inventors have founded that in the culturing process of reducing bacteria, a complex which is a compound of a metal reducing substance and a culture medium component has been obtained. Further, the inventors have founded that this complex has functioned as an adsorbent that quickly and efficiently adsorbs an ionic substance present in a solution, as well as functioned as a reducing agent that reduces specific precious metal ions or platinum-group metal ions and have completed the present invention. That is, in the present invention, a complex utilizing a metal reducing substance obtained in the culturing process of reducing bacteria and new functions thereof have been founded.

The complex of the present invention is a compound of a metal reducing substance and a culture medium component, which is produced in the culturing process of reducing bacteria. The reducing bacteria are bacteria having at least one biomineralization function.

It is preferable that the above-mentioned culture medium component contains an electron donor and an electron acceptor.

The above-mentioned reducing bacteria are iron-reducing bacteria and the iron-reducing bacteria belong to the genus Shewanella.

The above-mentioned complex contains phosphorus in mass ratio of about a quarter of the whole. The complex is a fibrous material or substantially spherical aggregation of the fibrous material in sub-micron diameter.

The above-mentioned complex has an adsorptive property and/or a reducing property.

The above-mentioned complex can be produced by a process of culturing reducing bacteria in a culture medium containing an oxide that can be reduced by the reducing bacteria; a process in which a reduced product obtained by the reducing bacteria and a component in the culture medium are bound; and a process in which the bound product is grown.

With the above-mentioned complex, ionic substances present in a solution can be adsorbed. It is preferable that this ionic substance is at least one metal selected from the group consisting of rare metal, precious metal, platinum-group metal, heavy metal and rare earth. In addition, by use of the complex, a precious metal ion or a platinum-group metal ion can be reduced.

### EFFECT OF THE INVENTION

The complex of the present invention is produced in the culturing process of reducing bacteria and is a compound of a metal reducing substance and a culture medium component. The reducing bacteria produce a metal reducing substance by being provided with a nutrient source (iron culture medium) only. This metal reducing substance and the culture medium component containing phosphorus in the culture media are bound to form a bound product. Further, the complex of the present invention can be stably stored at ordinary temperature for a relatively long time if being stored under wet condition.

Additionally, the obtained complex using the iron-reducing bacteria contains at least iron and phosphorus. This complex has a different function from conventional adsorbing molecules containing iron.

As such, the complex of the present invention has an adsorptive property and a reducing property. Therefore, the complex of the present invention can adsorbs ionic substances present in a solution rapidly and in good yield, or can reduce a precious metal ion or a platinum-group metal ion.

### BRIEF DESCRIPTION OF THE DRAWINGS

[Fig. 1] Fig. 1 is a graph showing the Dy adsorption rate of the complex according to the present invention.
[Fig. 2] Fig. 2 is a graph showing the Nd adsorption rate of the complex according to the present invention in which pH is changed.
[Fig. 3] Fig. 3 is a graph showing the each metal ion adsorption rate of the complex according to the present invention.
[Fig. 4] Fig. 4 is a graph evaluating a relation between the desorption amount of Dy and pH.
[Fig. 5] Fig. 5 is a graph evaluating the rare earth selectivity from a mixed solution.
[Fig. 6] Fig. 6 is a graph showing the Au adsorption rate of the complex according to the present invention.
[Fig. 7] Fig. 7 is a graph showing the Pt adsorption rate of the complex according to the present invention.
[Fig. 8] Fig. 8 is a graph showing the Rh adsorption rate of the complex according to the present invention.
[Fig. 9] Fig. 9 is a graph showing the Co adsorption rate of the complex according to the present invention.
[Fig. 10] Fig. 10 is a TEM photograph of the complex according to the present invention.
[Fig. 11] Fig. 11 is a graph showing the Pt adsorption rate of the complex prepared using a culture medium with the changed amount of potassium.

### BEST MODE FOR CARRYING OUT THE INVENTION

The present invention will be explained in detail in the following.

### [Complex]

The complex of the present invention is a complex produced in the culturing process of reducing bacteria and is a compound of a metal reducing substance and a culture medium component.

### (Reducing bacteria)

Any reducing bacteria having a biomineralization function can be used in the production of the complex of the present invention. The biomineralization function is a function of living organisms producing inorganic minerals. Examples of the reducing bacteria having the biomineralization function include known metal ion reducing bacteria such as iron-reducing bacteria and selenium reducing bacteria. The complex of the present invention is considered to be a compound of a metal reducing substance produced by reducing bacteria and a culture medium component. Therefore, any reducing bacteria being able to produce a metal reducing substance can be used. Among these reducing bacteria, iron-reducing bacteria are preferable. Examples of usable iron-reducing bacteria include, for example, ones shown below. The reducing bacteria may be used alone, or two or more kinds may be combined and used. In order to produce a uniform metal reducing substance, it is preferable to use single reducing bacterium alone.

### (Iron-reducing bacteria)

The iron-reducing bacteria used in the present invention are the bacteria which receive electrons from an electron donor and reduce iron. Examples of these iron-reducing bacteria include, for example, the genus Geobacter (representative: Geobacter metallireducens: strain ATCC (American Type Culture Collection) 53774), the genus Desulfuromonas (representative: Desulfuromonas palmitatis: strain ATCC 51701), the genus Desulfuromusa (representative: Desulfuromusa kysingii DSM (Deutsche Sammlung von Mikroorganismen und Zellkulturen) strain 7343), the genus Pelobacter (representative: Pelobacter venetianus: strain ATCC 2394), the genus Shewanella (Shewanella algae (hereinafter, referred to as "S. algae"): strain ATCC 51181, Shewanella oneidensis (hereinafter, referred to as "S. oneidensis"): strain ATCC 700550, Shewanella putrefacience (hereinafter, referred to as "S. putrefacience"): strain ATCC BAA-453), the genus Ferrimonas (Ferrimonas balearica: strain DSM 9799), the genus Aeromonas (Aeromonas hydrophila: strain ATCC 15467), the genus Sulfurospirillum (representative: Sulfurospirillum barnesii: strain ATCC 700032), the genus Wolinella (representative: Wolinella succinogenes: strain ATCC 29543), the genus Desulfovibrio (representative: Desulfovibrio desulfuricans: strain ATCC 29577), the genus Geothrix (representative: Geothrix fermentans: strain ATCC 700665), the genus Deferribacter (representative: Deferribacter thermophilus: strain DSM 14813), the genus Geovibrio (representative: Geovibrio ferrireducens: strain ATCC 51996), the genus Pyrobaculum islandicum: thermoproteus islandicum: strain DSM 4184), the genus Thermotoga (representative: Thermotoga maritima: strain DSM 3109), the genus Archaeoglobus (representative: Archaeoglobus fulgidus: strain ATCC 49558), the genus Pyrococcus (representative: Pyrococcus furiosus: strain ATCC 43587), the genus Pyrodictium (representative: Pyrodictium abyssi: strain DSM 6158). These iron-reducing bacteria are anaerobic bacteria.

The iron-reducing bacteria used in the present invention may conduct, by use of a culture media that is suitable for the bacteria, its growth and maintenance. For example, S. algae conduct a batch culture using, for example, a ferric citrate medium (ATCC No. 1931) having pH of 7.0 and in which sodium lactate (32 mol/m³) is contained as an electron donor and Fe(III) ion (56 mol/m³) is contained as an electron acceptor, to grow bacteria and maintain the same. Though the salt of iron ion is citrate in this example, it may be properly selected depending on the types of a culture medium or iron-reducing bacteria to be used. For example, examples of the electron donor include formate, hydrogen gas, lactate, pyruvate, acetate, benzoate, butyrate, citrate, ethanol, ethylene glycol, fructose, fumarate, glucose, glycerol, malate, phenol, succinate and tartrate. Examples of the electron acceptor include Mn(III), Mn(IV), Fe(III), Cr(VI) and U(VI).

Among the iron-reducing bacteria used in the present invention, iron-reducing bacteria of the genus Shewanella are preferable. Moreover, Shewanella algae, Shewanella oneidensis and Shewanella putrefacience are further preferable.

### (Production method of complex)

In the present invention, when the reducing bacteria to be used are cultured in a culture media in which the reducing bacteria causes a metal reduction reaction, a metal reducing substance that constitutes the complex of the present invention can be obtained. The metal reducing substance is produced, for example, as follows. In the following example, the case where iron-reducing bacteria are used to produce the complex will be explained. The same applies to the case where other reducing bacteria are used.

In the culture medium in which iron-reducing bacteria causes a metal reduction reaction, the culture and growth of bacteria is conducted at ordinary temperature and environment. In this culture medium, Fe(III) ion is generally contained as an electron acceptor. The iron-reducing bacteria oxidize lactic acid or citric acid contained in the culture medium to remove electrons, and grow. The removed electrons reduce the Fe(III) ion into Fe(II) ion. As a result, in the end stage of the growth after 24 to 36 hours of the culture, the culture medium in the above iron culture medium changes its color from black to brown. When the culture is further continued for about a week, the reduction amount of Fe(III) ion by bacteria is increased. In the culture medium, phosphate is contained. The Fe(II) ion obtained from the reduction reaction and phosphorus of the phosphate form a compound. In addition, a minor constituent is contained in the culture medium and this minor constituent may be incorporated into the complex. For example, by adding other constituents such as manganese or potassium into the culture medium, or otherwise increasing the amount of the addition constituent of the culture medium, the complex containing constituents other than iron and phosphorus can be obtained. The complex is precipitated in the bottom layer of the culture medium and as a result, the culture medium changes its color from brown to skin color.

The complex of the present invention contains phosphorus in mass ratio of about a quarter of the whole. If the complex mainly consists of iron and phosphorus, the contents of the iron and phosphorus is substantially 3:1 in mass ratio. If the complex further contains other constituents such as potassium or manganese, the content rate of the iron is decreased. By changing the content or type of other constituents contained in the culture medium, the function as an adsorbent or reducing agent can be changed. The function of the adsorbent or reducing agent is the types or amounts of substances to be adsorbed or reduced. Further, by changing the content or type of other constituents contained in the culture medium, a new function other than adsorbing or reducing function can be obtained. The constituents incorporated into the complex such as the above mentioned electron acceptor or potassium can be used for the other constituents contained in the culture medium. The potassium may be one which does not affect pH, such as potassium chloride, or one added as a pH adjuster, such as potassium hydroxide and potassium carbonate.

The complex of the present invention is a fibrous material or substantially spherical aggregation of the fibrous material in sub-micron diameter. As will be explained in Examples, a compound of a metal reducing substance and phosphorus or other constituents in the culture medium is initially fibrous. When the culture is continued, the complex becomes substantially spherical aggregation of the fibrous material in sub-micron diameter. The complex of the present invention has, no matter it is a fibrous material or substantially spherical aggregation of the fibrous material in sub-micron diameter, the similar function. Therefore, depending on the purpose of use, the shape thereof may be selected to be used. Further, since the complex of the present invention is a fibrous material or substantially spherical aggregation of the fibrous material in sub-micron diameter, the reaction surface area is very large and as a result, the complex exercises an excellent processing efficiency when used as an adsorbent or reducing agent.

The complex of the present invention can be obtained by a solid-liquid separation of the cultured culture medium using a known separation method such as centrifugal separation. To the separated solids is added ion-exchange water or distilled water or the like, followed by washing by stirring and mixing. Then the solids are again recovered using centrifugal separation and the like and a small amount of ion-exchange water or distilled water is poured in the solids for state maintenance and the mixture is allowed to stand.

The above solids are a mixture of the complex and bacteria. If the mixture is left at room temperature for a few days with a small amount of ion-exchange water or distilled water being poured in the solids, the solids are separated into a bacteria layer (supernatant) and a complex layer (lower layer). By discarding the supernatant bacteria layer, the complex can be obtained. These treatments can be conducted under aerobic condition even if the iron-reducing bacteria to be used are anaerobic bacteria. The obtained complex can be stored at room temperature if stored under conditions preventing water evaporation (for example, stored in a bottle with a lid). In this condition, the adsorbing capability or reducing capability can be maintained, even after a few weeks.

The obtaining complexes are different depending on the type of reducing bacteria to be used. However, in case where a subject to be reduced by the reducing bacteria is common, such as iron, the reduction state is common (for example, having the same reduced valance) and similar culture media are used, similar complexes can be obtained. For example, when S. algae, S. oneidensis and S. putrefacience are used, similar fibrous materials or substantially spherical aggregation of the fibrous materials in sub-micron diameter can be obtained. They are also common in that the compositions of the complexes include iron and phosphorus. The substantially spherical aggregation of the fibrous material means, as used herein, that the material may be spherical or may have a deficit in the part of the sphere. In the case where the iron-reducing bacteria are the genus Shewanella, the complex is a fibrous material or substantially spherical aggregation of the fibrous material in sub-micron diameter. Many of the substantially spherical aggregation have a particle diameter of 100 nm to 1 µm and some has a particle diameter of about 2 µm and a mean particle diameter is about a sub-micron size. Moreover, the periphery of the complex has more phosphorus than the inside thereof and has an adsorbing function.

### (Adsorbing function of complex)

The complex of the present invention is adsorptive. The complex of the present invention can adsorb ionic substances present in a solution. The ionic substance present in the solution may be an organic substance or an inorganic substance. It is preferable if the ionic substance is a metal. Examples of the metals to be adsorbed include, for example, rare metals, precious metals, platinum-group metals, heavy metals and rare earth. Elements included in Rare metals are partially common to elements included in platinum-group metals. The ionic substance to be subjected to the adsorption treatment may be one type or multiple types. In addition, by containing other constituents than iron and phosphorus as a constituent of the complex, the substance to be adsorbed or the adsorption amount can be properly changed.

Examples of rare metals include lithium, beryllium, boron, titanium, vanadium, chromium, manganese, cobalt, nickel, gallium, germanium, selenium, rubidium, strontium, zirconium, niobium, molybdenum, palladium, indium, antimony, tellurium, cesium, barium, hafnium, tantalum, tungsten, rhenium, platinum, thallium and bismuth.

Examples of precious metals include gold, silver, platinum, palladium, rhodium, iridium, ruthenium and osmium.

Examples of platinum-group metals include ruthenium, rhodium, palladium, osmium, iridium and platinum.

Examples of heavy metals include copper, chromium, cadmium, mercury, zinc, arsenic, manganese, cobalt, nickel, molybdenum, tungsten, tin, bismuth, uranium and plutonium.

Rare earth is a rare-earth element and examples thereof include scandium, yttrium, lanthanum, cerium, praseodymium, neodymium, promethium, samarium, europium, gadolinium, terbium, dysprosium, holmium, erbium, thulium, ytterbium and lutetium.

The complex of the present invention can adsorb ionic substances present in a solution within a wide pH range of acidic to alkaline solution. For example, the pH range is about pH 1.0 to 10.0. As such, the complex of the present invention can adsorb ionic substances present in a solution within a wide pH range from weak alkaline condition to neutral condition and to strongly acidic condition. Further, by adjusting the pH of the adsorbent after having adsorbed the ionic substances such that the adsorbent is under strongly acidic condition lower than the above adsorption pH, the adsorbed ionic substances can be desorbed.

With the use of the complex of the present invention, heavy metals contained in wastewater can be effectively removed irrespective of the valance of metals or pH of a process liquid. Also, the complex of the invention can easily adsorb rare metals, precious metals, platinum-group metals, rare earth and the like contained in electronic materials, electronic devices and minerals by letting them into a solution state. Therefore, rare metals, precious metals, platinum-group metals, rare earth and the like can be easily recovered. Further, the complex of the invention can easily adsorb metals. Therefore, the complex adsorbing ionic substances can be directly used for an active material of battery and the like.

The complex of the invention adsorbs ionic substances by an ion-exchange reaction. Therefore, after the ionic substances are desorbed, the complex can be regenerated. Additionally, the complex of the invention has different adsorptive properties depending on the ionic substances. Therefore, if multiple ionic substances present, the complex can selectively adsorb. As such, the complex of the invention has different functions. As a result, by selecting adsorption conditions and desorption conditions, individual materials can be separated from the plural ionic substances in good purity.

### (Reducing function of complex)

The complex of the invention has a reducing property. The complex of the invention can reduce ionic substances present in a solution. The complex mainly consisting of iron and phosphorus reduces, for example, precious metals and platinum-group metals such as gold, platinum and palladium. Further, by containing constituents other than iron and phosphorus as constituents of the complex, the material to be reduced and the reduction amount can be properly changed.

The complex of the invention can maintain its functions such as adsorbing capability and reducing capability at room temperature for a few or more weeks if being stored under wet condition.

### EXAMPLE

The present invention will be explained below using Examples, but the invention is not limited thereto.

### (Example 1)

### (Preparation of complex)

Iron-reducing bacteria S. algae (strain ATCC 51181) were cultivated and grown in a ferric citrate culture medium (ATCC No. 1931). The culture medium contains 56 mol/m³ of Fe(III) ion as an electron acceptor. In the end stage of the growth of the bacteria, the Fe(III) ion in the iron culture medium was reduced by the bacteria and the culture medium changed its color from black to brown.

When the culture is further continued for about a week, the amount of the Fe(III) which was reduced by the bacteria was increased and the culture medium changed its color from brown to skin color. The reduced material was precipitated in the bottom layer of the culture medium. The cultured culture medium was subjected a solid-liquid separation using centrifugal separation (8000 rpm) to recover the precipitate. Into the precipitate was poured ion-exchange water for washing and the mixture was stirred, followed by centrifugal separation to recover the precipitate. A small amount of ion-exchange water was poured in this precipitate.

The obtained precipitate was allowed to stand for a few days at room temperature. The precipitate was separated into a bacteria layer (supernatant) and a reduced product layer (lower layer). The supernatant bacteria layer was discarded to obtain a complex.

### (Example 2)

### (Rare earth (Dy) recovery performance)

By use of the complex prepared in the above production method, the recovery performance on rare-earth elements was evaluated.

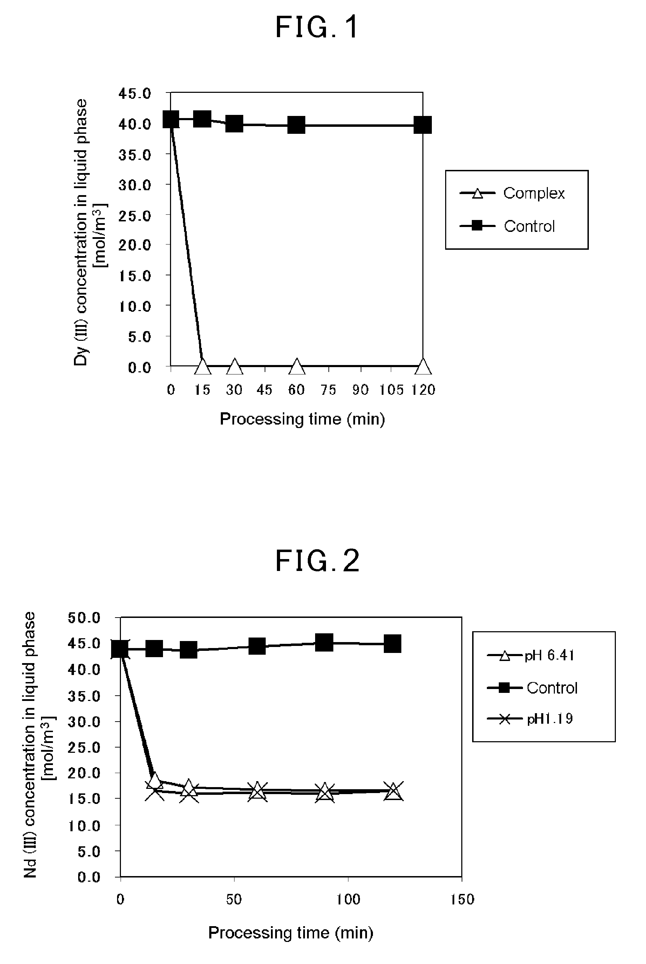

The dysprosium chloride (DyCL₃) solution in which the concentration thereof has been adjusted was used as a sample. Into 1.4 mL of the dysprosium chloride solution was poured 5 ml of the above complex diluted in fivefold dilution. A process liquid was adjusted such that the total volume became 6.4 ml and the final concentration of dysprosium(III) became 40 mol/m³. Stirring the liquid, 500 µL of the liquid was collected at each lapse time and the concentration of Dy in the solution in which the complex was separated by centrifugal separation was measured using ICP (inductively coupled plasma) emission analyzer. The solution of the same concentration in which the complex was not added was also collected and the concentration was measured as a control. All operations were conducted under room temperature condition and aerobic condition. The results are shown in Fig. 1. Fig. 1 is a graph showing the Dy adsorption rate of the complex according to the invention. In Fig. 1, the abscissa represents processing time (minute ("min" in the drawing)) and the ordinate represents a concentration of dysprosium(III) (mol/m³) dissolved in the process liquid. Further, the symbol ■ represents in the case where the complex was not added and the symbol Δ represents in the case where the complex was added, respectively.

From Fig. 1, it can be found that by use of the complex of this Example, Dy does not exist in the solution after 15 minutes from the initiation of reaction. Therefore, it can be recognized that by use of the complex of this Example, dysprosium(III) of 40 mol/m³ can be 100% adsorbed within 15 minutes from the initiation of reaction. That is, it can be recognized that the complex of this Example can function as an adsorbent. Moreover, the saturated adsorption amount of dysprosium (Dy) in the complex was 0.3 to 0.48 g/g.

### (Example 3)

### (Adsorption evaluation under acidic condition)

By use of the complex prepared in the above production method (Example 1), the adsorption under acidic condition was evaluated.

The neodymium chloride (NdCl₃) solution in which the concentration thereof has been adjusted was used as a sample. 2 mL of the complex of Example 1 diluted in fivefold dilution was poured thereto. A process liquid was adjusted such that the total volume became 7 mL and the final concentration became 44 mM (Nd). Under the above concentration condition, with respect to two samples each having pH of 1.19 (after the complex was added: pH of 2.17) and pH of 6.41 (after the complex was added: pH of 4.18), pH effect on the recovery rate was evaluated. During the reaction, 500 µL of the liquid was obtained at each lapse time and the complex was separated using centrifugal separation. The concentration of Nd in the solution after separation was measured using ICP emission analyzer. The solution of the same concentration in which the complex was not added was also obtained and the concentration was measured as a control. All operations were conducted under room temperature condition and aerobic condition. The results are shown in Fig. 2. Fig. 2 is a graph showing the Nd adsorption rate of the complex according to the invention as changing pH. In Fig. 2, the abscissa represents processing time (minute ("min" in the drawing)) and the ordinate represents a concentration of Nd(III) (mol/m³) dissolved in the process liquid. Further, the symbol ■ represents in the case where the complex was not added; the symbol x represents in the case where the complex was added at pH of 1.19; and the symbol Δ represents in the case where the complex was added at pH of 6.41, respectively.

From Fig. 2, it can be found that by use of the complex of this Example, even if pH is adjusted to either pH of 1.9 (after the complex was added: pH of 2.17) or pH of 6.41 (after the complex was added: pH of 4.18), there is no significant difference in the Nd adsorption rate. Therefore, it can be recognized that by use of the complex of this Example, adsorption is possible even under acidic conditions. It can be also recognized that the complex of this Example functions as an adsorbent.

Moreover, since it is possible to adsorb not only dysprosium(III) used in Example 2 but also neodymium, it can be recognized that various rare earth can be adsorbed. Moreover, the saturated adsorption amount of neodymium (Nd) in the complex was 0.25 to 0.35 g/g.

### (Example 4)

### (Application to various metal elements and Evaluation of selectivity thereto)

By use of the complex prepared in the above production method, the adsorptive property and selectivity for Pd (PdCl₃), In (InCl₃) and Ga (GaCl₃) were evaluated.

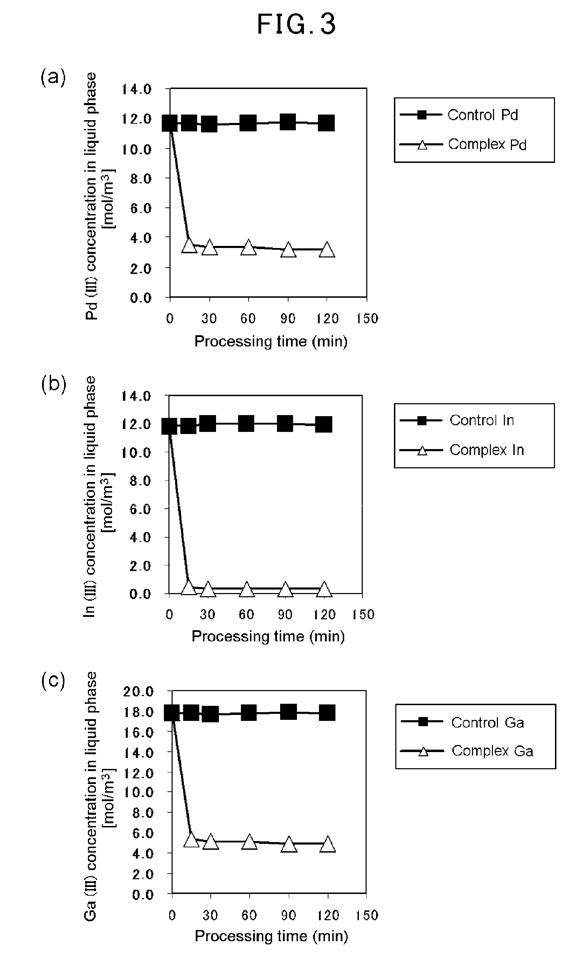

The Pd (PdCl₃, metal concentration: 11.7 mM, the total reaction volume: 5 mL) solution, In (InCl₃, metal concentration: 11.8 mM, the total reaction volume: 5 mL) solution and Ga (GaCl₃, metal concentration: 17.8 mM, the total reaction volume: 5 mL) solution in which the concentrations thereof have been adjusted were prepared as a sample. The complex obtained in Example 1 was diluted in fivefold dilution and 2 mL thereof was poured to determine a metal ion recovery rate. During the reaction, 500 µL of the liquid was collected at each lapse time and the complex was separated using centrifugal separation. The concentration of each metal ion of the obtained solutions was measured using ICP emission analyzer. The solution of the same concentration in which the complex was not added was also collected and the concentration was measured as a control. All operations were conducted under room temperature condition and aerobic condition. The results are shown in Fig. 3. Fig. 3 is a graph showing the each metal ion adsorption rate of the complex according to the invention. Fig. 3(a) shows the Pd adsorption rate; Fig. 3(b) shows the In adsorption rate; and Fig. 3(c) shows the Ga adsorption rate. In Fig. 3, the abscissa represents processing time (minute ("min" in the drawing)) and the ordinate represents a concentration of Pd(III), concentration of In(III) and concentration of Ga(III) (mol/m³) dissolved in the process liquid. Further, the symbol ■ represents in the case where the complex was not added and the symbol Δ represents in the cases of Pd, In and Ga, respectively.

From Fig. 3, it can be recognized that the complex of this Example can recover the three elements of Pd, In and Ga. The complex of the same concentration was used for each recovery test. When In is compared with Pd having substantially the same concentration and volume, it can be recognized that in the case of In ion the comoplex represents higher adsorptive property than those in the case of Pd ion and thus this complex has adsorption selectivity. Further, in the Pd ion recovery reaction, a color change which indicates a metal reduction was found and fine particles were formed. From this, it can be recognized that this complex has reducing function. Moreover, the saturated adsorption amount of palladium (Pd) in the complex was 0.12 to 0.18 g/g.

Further, in the adsorption reaction of palladium of this Example, a color change which indicates a reduction reaction was observed immediately after mixing the metal solution and the complex. The solution after reaction was subjected to X-ray-absorption fine-structure spectroscopy (XAFS) using a large scale radiation facility (TOYOTA Beamline: BL33XU) and it was found that the Pd(II) has been reduced into Pd(O). Therefore, this complex has not only a function of adsorbing Pd, but also a function of reducing Pd.

### (Example 5)

### (Evaluation of desorption and collection of adsorbed ion)

By use of the complex prepared in the above production method (Example 1), Dy was recovered from the dysprosium chloride (DyCL₃) solution and the desorption and collection of metal ion from the reduction material after recovery were evaluated.

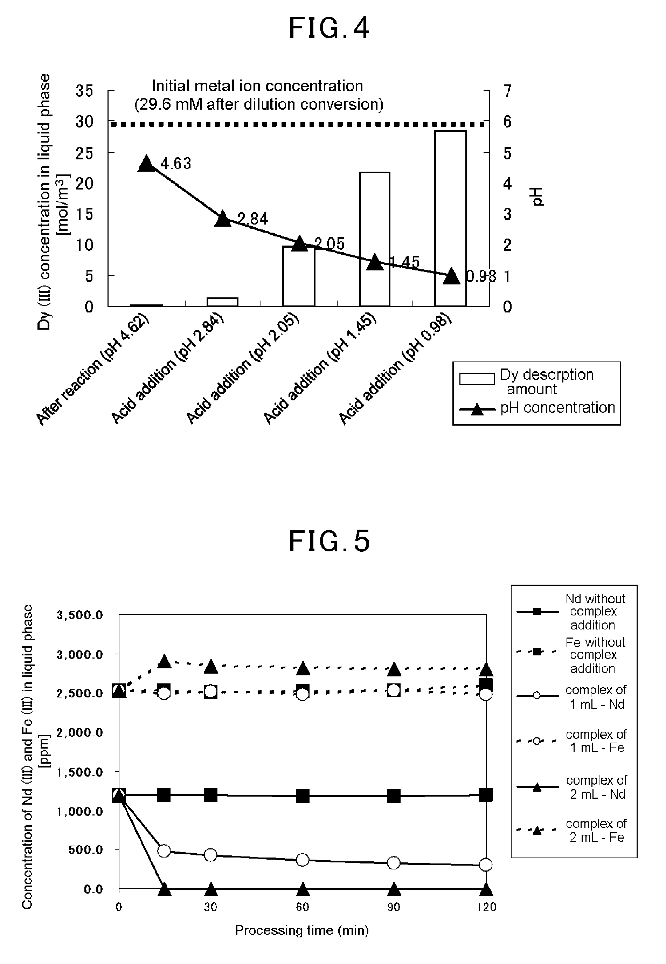

The Dy solution in which the concentration thereof has been adjusted (DyCl₃, metal concentration: 34.4 mM, the total reaction volume: 7.5 mL) was prepared as a sample. To this solution was poured 1.5 mL of the complex of Example 1 diluted in 5-fold dilution and the all amount of Dy (DyCL₃) was recovered in a two-hour reaction (confirmed using ICP emission analyzer). To 1 mL aliquots of this liquid was added 160 µL of the HCI solutions in which the concentrations thereof were adjusted to different pH, respectively. The pH of the liquid after the HCL solution was added was 2.84, 2.05, 1.45 and 0.98, respectively. Each liquid was stirred for 30 minutes. Then the concentration of the metal in the solution after separation of the complex by centrifugal separation was measured using ICP emission analyzer. From that, the relation between the desorption amount of Dy and pH was evaluated. The results are shown in Fig. 4. Fig. 4 is a graph evaluating a relation between the desorption amount of Dy and pH. In Fig. 4, the abscissa represents pH of the liquid after the HCL solution was added and the ordinates represent a concentration of Dy(III) in the liquid phase and desorption amount of Dy, respectively.

From Fig. 4, it can be recognized that the desorption amount of Dy is increased as the pH condition becomes more acidic. The desorption amount is 80% or more at pH of 1.4 and reaches over 95% at pH of 0.98. Therefore, it can be recognized that the metal recovered by the complex can be desorbed and collected again by adjusting the pH.

### (Example 6)

### (Rare earth selectivity from mixed solution)

By use of the complex prepared in the above production method (Example 1), applicability to the recovery of rare earth (Nd) from a mixed solution of Fe (FeCl₃) and Nd (NdCl₃) was evaluated.

The sample in which the concentration thereof has been adjusted (Fe: 2500 ppm, Nd: 1100 ppm) such that a blend ratio of the elements becomes the one anticipating neodymium magnets (Nd: 30%, Fe: 68%) was prepared. The complex of Example 1 and ion-exchange water were added thereto and a recovery reaction was conducted with the total volume of 5 mL. The input of the complex was poured in two levels i.e. in 1 mL and 2mL and the influence on the metal ion recovering behavior of the complex was also examined. During the reaction, 500 µL of the liquid was collected at each lapse time. The complex in this liquid was separated using centrifugal separation and the concentration of each metal ion in the solution was measured using ICP emission analyzer. The solution of the same concentration in which the complex was not added was also collected and the concentration thereof was measured as a control. All operations were conducted under room temperature condition and aerobic condition. The results are shown in Fig. 5. Fig. 5 is a graph evaluating rare earth selectivity from the mixed solution.

From Fig. 5, it can be recognized that the recovered amount of the Nd element increases in proportion to the input of the complex (1 mL, 2 mL). On the other hand, the Fe concentration did not change when the input was 1 mL and increased when the input was 2 mL. Since the recovery reaction of the complex of the invention utilizes an ion-exchange reaction, it is assumed that the Fe in the complex is released in connection with the adsorption of the Nd element. In any event, it can be recognized that this complex has high Nd selectivity even in the mixed solution.

### (Example 7)

### (Evaluation of recovery performance on Au)

By use of the complex prepared in the above production method (Example 1), the recovery performance on Au(III) was experimentally examined.

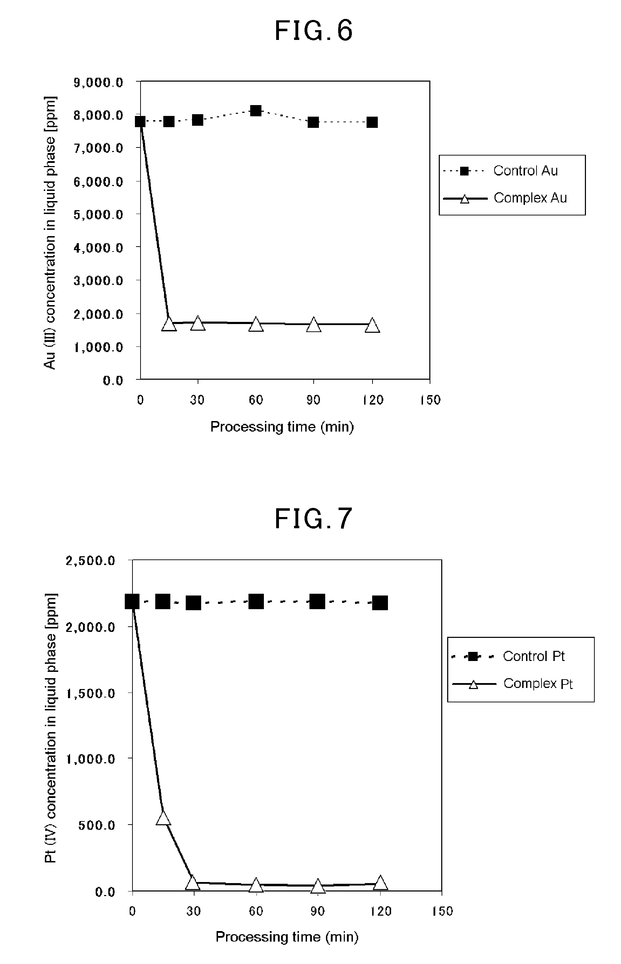

The gold chloride solution (AuCl₃) in which the concentration thereof has been adjusted was used as a sample. To 2.6 mL of the sample was added 0.5 mL of the complex solution (complex mass (it means "the amount of the complex contained in the complex solution" and the same definition applies hereinafter): 0.075 g) and the reaction was conducted in the total volume of 3.1 mL. During the reaction, a reaction liquid was stirred with a rotor. The reaction liquid was collected by 500 µL at each lapse time and the complex was separated by centrifugal separation. The concentration of the metal (Au) in the separated solution was measured using ICP (inductively coupled plasma) emission analyzer. The solution of the same concentration in which ion-exchange water was added instead of the complex was also collected and measured in the same manner and which was used as an initial control. All operations were conducted under room temperature condition and aerobic condition. The results are shown in Fig. 6. Fig. 6 is a graph showing Au adsorption rate of the complex according to the invention. In Fig. 6, the abscissa represents processing time (minute ("min" in the drawing)) and the ordinate represents a concentration of gold(III) (ppm) dissolved in the process liquid. Further, the symbol ■ represents in the case where the complex was not added and the symbol Δ represents in the case where the complex was added, respectively.

From Fig. 6, it can be recognized that in the sample collected after 15 minutes of the reaction in which the complex is mixed into the solution having the initial concentration of Au of 7800 ppm, the concentration of Au is decreased to 1700 ppm. From this, the complex of the invention is recognized to have a property adsorbing Au. Additionally, the saturated adsorption amount calculated from the metal concentration and the complex amount was 0.21 g/g Dry.

Further, in the adsorption reaction of this Example, a color change indicating a reduction reaction was observed immediately after mixing the metal solution and the complex. The solution after reaction was subjected to X-ray-absorption fine-structure spectroscopy (XAFS) using a large scale radiation facility (TOYOTA Beamline: BL33XU) and it was found that the Au(III) has been reduced into Au(0). Therefore, this complex has not only a function of adsorbing Au, but also a function of reducing Au.

### (Example 8)

### (Evaluation of recovery performance on Pt)

By use of the complex prepared in the above production method (Example 1), the recovery performance on Pt(IV) was experimentally examined.

The hexachloroplatinic(IV) acid solution (H₂PtCl₆) in which the concentration thereof has been adjusted was used as a sample. To 2.8 mL of the sample was added 0.5 mL of the complex solution (complex mass: 0.089 g) and the reaction was conducted in the total volume of 3.3 mL. During the reaction, a reaction liquid was stirred with a rotor. The reaction liquid was collected by 500 µL at each lapse time and the complex was separated by centrifugal separation. The concentration of the metal (Pt) in the separated solution was measured using ICP (inductively coupled plasma) emission analyzer. The solution of the same concentration in which ion-exchange water was added instead of the complex was also collected and measured in the same manner and which was used as an initial control. All operations were conducted under room temperature condition and aerobic condition. The results are shown in Fig. 7. Fig. 7 is a graph showing the Pt adsorption rate of the complex according to the invention. In Fig. 7, the abscissa represents processing time (minute ("min" in the drawing)) and the ordinate represents a concentration of platinum (IV) (ppm) dissolved in the process liquid. Further, the symbol ■ represents in the case where the complex was not added and the symbol Δ represents in the case where the complex was added, respectively.

From Fig. 7, it can be found that in the sample collected after 30 minutes of the reaction in which the complex is mixed into the solution having the initial Pt concentration of 2200 ppm, the concentration of Pt is decreased to 550 ppm. From this, the complex of the invention is recognized to have a property adsorbing Pt. Additionally, the saturated adsorption amount calculated from the metal concentration and the complex amount was 0.08 g/g Dry.

Further, in the adsorption reaction of this Example, a color change indicating a reduction state was observed after mixing the metal solution and the complex. The solution after the reaction was subjected to X-ray-absorption fine-structure spectroscopy (XAFS) using a large scale radiation facility (TOYOTA Beamline: BL33XU) and it was found that the Pt(IV) has been reduced into Pt(O). Therefore, this complex has not only a function adsorbing Pt, but also a function reducing Pt.

### (Example 9)

### (Evaluation of recovery performance on Rh)

By use of the complex prepared in the above production method (Example 1), the recovery performance on Rh(III) was experimentally examined.

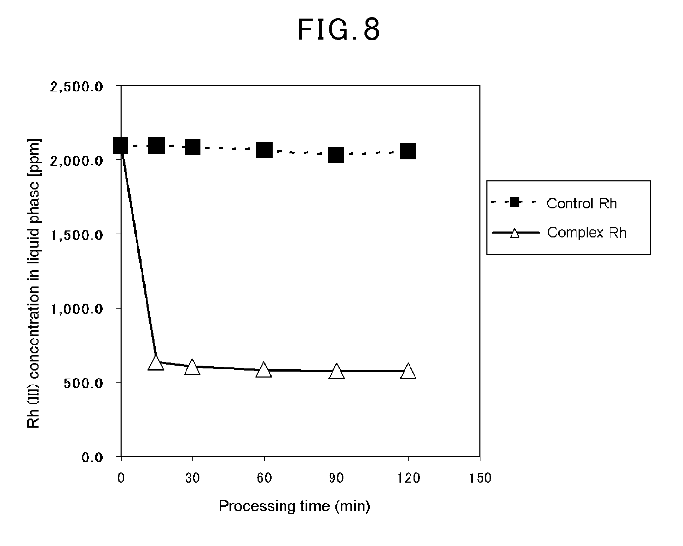

The rhodium(III) chloride solution (RhCl₃) in which the concentration thereof has been adjusted was used as a sample. To 3 mL of the sample was added 0.5 mL of the complex solution (complex mass: 0.106 g) and the reaction was conducted in the total volume of 3.5 mL. During the reaction, a reaction liquid was stirred with a rotor. The reaction liquid was collected by 500 µL at each lapse time and the complex was separated by centrifugal separation. The concentration of the metal (Rh) in the separated solution was measured using ICP (inductively coupled plasma) emission analyzer. The solution of the same concentration in which ion-exchange water was added instead of the complex was also collected and measured in the same manner and which was used as an initial control. All operations were conducted under room temperature condition and aerobic condition. The results are shown in Fig. 8. Fig. 8 is a graph showing the Rh adsorption rate of the complex according to the invention. In Fig. 8, the abscissa represents processing time (minute ("min" in the drawing)) and the ordinate represents a concentration of rhodium(III) (ppm) dissolved in the process liquid. Further, the symbol ■ represents in the case where the complex was not added and the symbol Δ represents in the case where the complex was added, respectively.

From Fig. 8, it can be recognized that in the sample collected after 15 minutes of the reaction in which the complex is mixed into the solution having the initial Rh concentration of 2060 ppm, the Rh concentration is decreased to 630 ppm. From this, the complex of the invention is recognized to have a property adsorbing Rh. Additionally, the saturated adsorption amount calculated from the metal concentration and the complex amount was 0.05 g/g Dry.

### (Example 10)

### (Evaluation of recovery performance on Co)

By use of the complex prepared in the above production method (Example 1), the recovery performance on Co(III) was experimentally examined.

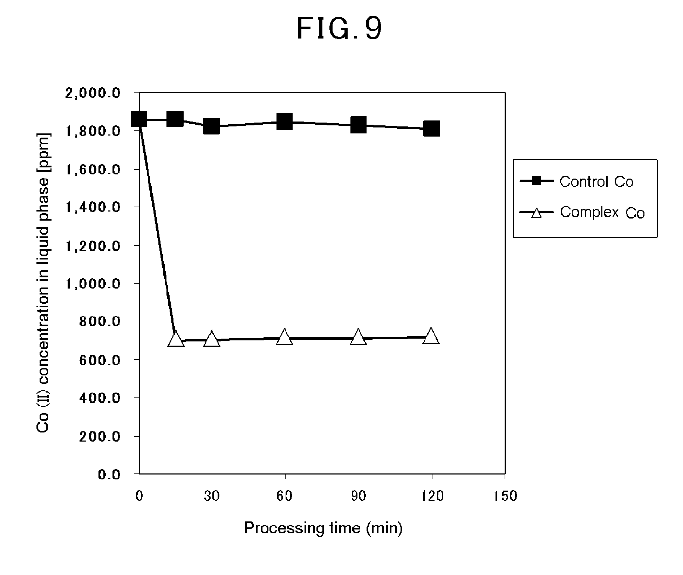

The cobalt chloride solution (CoCl₂) in which the concentration thereof has been adjusted was used as a sample. To 2 mL of the sample was added 1.0 mL of the complex solution (complex mass: 0.22 g) and the reaction was conducted in the total volume of 3.0 mL. During the reaction, a reaction liquid was stirred with a rotor. The reaction liquid was collected by 500 µL at each lapse time and the complex was separated by centrifugal separation. The concentration of the metal (Co) in the separated solution was measured using ICP (inductively coupled plasma) emission analyzer. The solution of the same concentration in which ion-exchange water was added instead of the complex was also collected and measured in the same manner and which was used as an initial control. All operations were conducted under room temperature condition and aerobic condition. The results are shown in Fig. 9. Fig. 9 is a graph showing the Co adsorption rate of the complex according to the invention. In Fig. 9, the abscissa represents processing time (minute ("min" in the drawing)) and the ordinate represents a concentration of cobalt(II) (ppm) dissolved in the process liquid. Further, the symbol ■ represents in the case where the complex was not added and the symbol Δ represents in the case where the complex was added, respectively.

From Fig. 9, it can be recognized that in the sample collected after 15 minutes of the reaction in which the complex is mixed into the solution having the initial Co concentration of 1860 ppm, the concentration of Co is decreased to 700 ppm. From this, the complex of the invention is recognized to have a property adsorbing Co. Additionally, the saturated adsorption amount calculated from the metal concentration and the complex amount was 0.02 g/g Dry.

### (Example 11)

### (Structure analysis of complex)

The fine inner structure of the complex of the present invention was analyzed in detain using a transmission electron microscope (TEM JEM-2100F manufactured by JOEL, Ltd.).

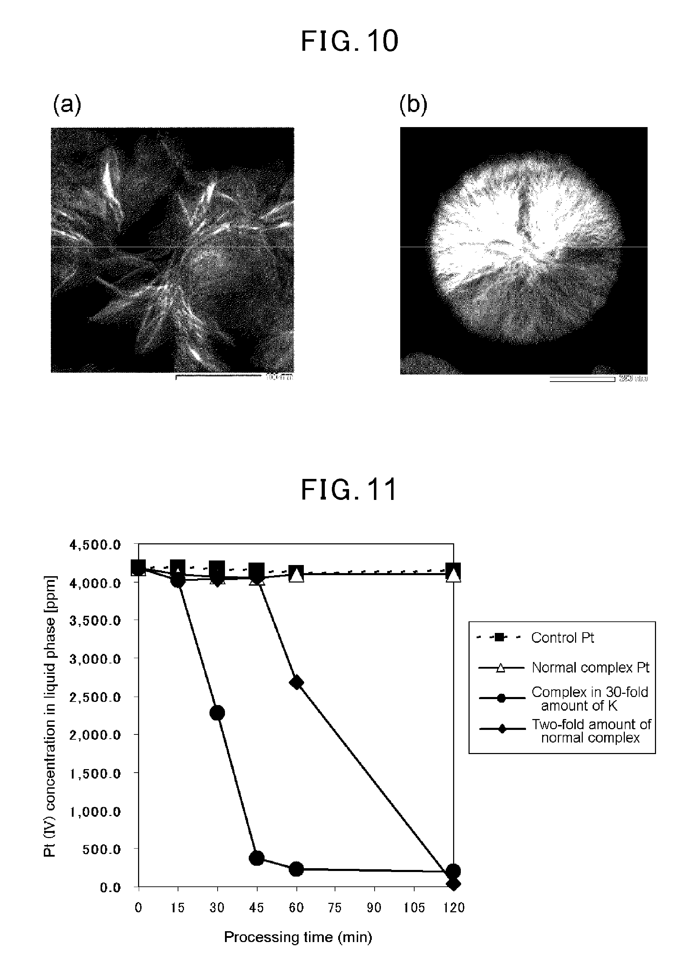

The non-spherical complex produced in the initial state of the culture and complex which became substantially spherical form with the development of the culture were used. The complex in the form of aqueous solution was subjected to acetone substitution and was substituted and fixed by an epoxy resin, followed by preparation of a section of 70 nm with a microtome. The fine inner structure of the complexes being adsorbent is analyzed using a transmission electron microscope. The results are shown in Fig. 10. Fig. 10 is a photograph of the complex according to the invention, which is taken using TEM. Fig. 10(a) shows the non-spherical complexes produced in the initial state of the culture and Fig. 10(b) shows the complex which became substantially spherical form with the development of the culture.

From Fig. 10, it can be recognized that the complex of the invention is fibrous and the complex becomes the structure where the fibrous complexes are substantially spherically aggregated as the culture develops. Further, since the complex of the invention is aggregation of the fibrous complexes, it is recognized that the reaction surface area is large.

It was also confirmed that even by use of the fibrous complex in the initial state of the culture, the complex had an adsorption capability toward rare earth and the like.

### (Example 12)

### (Consideration of complex preparation capability of reducing bacteria)

By use of three types of bacteria: S. algae, S. oneidensis and S. putrefaciens, a culture was conducted in the same iron culture medium as that of Example 1, followed by consideration of availability of complex preparation and experimental consideration regarding adsorption capability toward a metal ion.

It was found that the similar complexes could be prepared from the three types of bacteria cultured under the same culture media conditions. It was confirmed that each complex exhibited an adsorption reaction toward various metal ions (Nd, Dy, Au, Pt, Pd, Rh) without significant difference. Table 1 shows the saturated adsorption amount of each element in each complex.

[Table 1]

**TABLE 1**

| | | | Unit: g/g |
|---|---|---|---|
| Element | Species of Bacteria for complex | | |
| | S. algae | S. oneidensis | S. putrefaciens |
| Nd | 0.27 | 0.28 | 0.28 |
| Dy | 0.33 | 0.32 | 0.32 |
| Au | 0.19 | 0.18 | 0.2 |
| Pd | 0.14 | 0.14 | 0.14 |
| Pt | 0.08 | 0.08 | 0.08 |
| Rh | 0.04 | 0.04 | 0.05 |

From Table 1, it is recognized that the complexes prepared by each bacteria have a similar adsorptive property toward different metals. Further, the complexes had a reducing property toward Au, Pd and Pt. From this, it is recognized that by use of the metal-reducing bacteria having an iron-reducing function, similar complexes can be obtained.

### (Example 13)

### (Consideration of change in composition of adsorbent due to composition modification of culture medium)

The complex obtained in Example 1 was dissolved in 2M-HCl and an element analysis was performed using an ICP (inductively coupled plasma) emission analyzer. It was found that a plurality of trace elements was contained in the complex in addition to the main constituents of Fe and P. These elements were assumed to be derived from the constituents contained in the culture medium in minute amounts. It was found that one of the minor constituents was manganese (Mn). This manganese was assumed to be derived from manganese sulfate (MnSO₄) contained in the culture medium in the amount of 1 mg/L.

The culture media in which the amount of manganese sulfate in the culture medium was increased 100-fold, 1000-fold and 2000-fold were prepared and a culture was conducted using S. algae to prepare complexes. Each complex was dissolved in HCl and an element analysis was performed using an ICP (inductively coupled plasma) emission analyzer. The results are shown in Table 2.

[Table 2]

**TABLE 2**

| | Fe | P | Mn |
|---|---|---|---|
| Complex prepared by usual culture medium | 75.5% | 23.4% | 0.5% |
| Complex MnSO₄-100-fold added | 73.1% | 23.8% | 2.6% |
| Complex MnSO₄-1000-fold added | 59.4% | 24.1% | 15.6% |
| Complex MnSO₄-2000-fold added | 50.7% | 23.5% | 25.1% |

From Table 2, it is recognized that the Mn ratio in the complex increases in proportion to the increased amount of MnSO₄ added into the culture medium. In addition, the Fe ratio was decreased. On the other hand, it was found that there was no great change in the P ratio in each complexes and P accounted for about a quarter thereof. From these results, it can be recognized that the complex in which the amount of phosphorus is about a quarter and other compositions are different can be obtained by modifying the constituents of the culture medium.

### (Example 14)

### (Metal adsorbing effect of complex prepared by composition modification of culture medium)

The amount of potassium chloride (KCI), which was usually contained in the culture medium in the amount of 0.1 g/L, increased 30-fold (3 g/L) and S. algae was cultured to prepare a complex. The recovery performance of this complex on Pt was compared with that of the usually prepared complex.

The hexachloroplatinic(IV) acid solution (H₂PtCl₆) in which the concentration thereof have been adjusted was used as a sample. The reactivity was compared among three samples: (1) sample in which 0.5 mL (adsorbent content: 0.14 g) of the complex solution prepared in the usual culture medium was added to 2.0 mL of the sample and 0.5 mL of ion-exchange water was further added thereto, having the total volume of 3.0 mL; (2) sample in which 0.5 mL (adsorbent content: 0.14 g) of the complex prepared in the usual culture medium containing 30-fold amount of KCl, was added and 0.5 mL of ion-exchange water was further added, having the total volume of 3.0 mL; and (3) sample in which the amount of the complex prepared in the usual culture medium was increased 2-fold (1.0 mL, adsorbent content: 0.28 g) and ion-exchange water was not added, having the total volume of 3.0 mL. During the reaction, a reaction liquid was stirred with a rotor. The reaction liquid was collected by 500 µL at each lapse time and the adsorbent was separated by centrifugal separation. The concentration of the metal (Pt) in the separated solution was measured using ICP (inductively coupled plasma) emission analyzer. The solution of the same concentration in which ion-exchange water was added instead of the complex was also collected and measured in the same manner and which was used as an initial control. All operations were conducted under room temperature condition and aerobic condition. The results are shown in Fig. 11. Fig. 11 is a graph showing the Pt adsorption rate of the complex according to the invention prepared using a culture medium with changing the amount of potassium. In Fig. 11, the abscissa represents processing time (minute ("min" in the drawing)) and the ordinate represents a concentration of Pt(IV) (ppm) dissolved in the process liquid. Further, the symbol ■ represents in the case where the complex was not added; the symbol Δ represents in the case where the complex prepared using the usual culture medium was added; the symbol ● represents in the case where the complex prepared in the usual culture medium containing the 30-fold amount of KCl was added; and the symbol ◆ represents in the case where the twofold amount of the complex prepared using the usual culture media was added, respectively.

As can be recognized from Fig. 11, in the sample in which the complex prepared using the usual culture medium is mixed into the solution having the initial Pt concentration of 4200 ppm, a reaction can be hardly seen due to the high initial concentration of metal. The complex prepared in the culture medium where the amount of KCl was increased 30-fold reacts for 15 to 45 minutes and reaches a saturation point after 60 minutes. The both reactions use the same amount of complex and it can be recognized that the complex prepared in the culture medium where the amount of KCl was increased 30-fold has more excellent reactivity. Moreover, in comparison of this complex with the complex in which the amount of the complex prepared in the usual culture media was increased twofold, the reaction initiation time is clearly different and it can be recognized that the complex prepared in the culture medium where the amount of KCl was increased 30-fold is more excellent also in rapidity.

As such, it can be recognized that in the complex of the present invention, the complex composition is changed by modifying the culture medium composition and this modification affects a metal ion adsorbing reaction. Therefore, it is recognized that the complexes with different characters can be prepared by composition modification depending adjusted to a target metal ion.

### INDUSTRIAL APPLICABILITY

With the use of the complex of the present invention, heavy metals contained in wastewater can be effectively removed irrespective of the valance of metals or pH of a process liquid. Also, the complex of the invention can easily adsorb on rare metals, precious metals, platinum-group metals, rare earth and the like contained in electronic materials, electronic devices and minerals by letting them into a solution state. Therefore, rare metals, precious metals, platinum-group metals, rare earth and the like can be easily recovered. Further, the complex of the invention can easily adsorb on metals. Therefore, the complex adsorbing ionic substances can be directly used for an active material of battery and the like. In addition, since the complex of the invention can reduce specific precious metals and platinum-group metals, the complex can be used as a novel reducing agent.

## Claims

1. A complex being a compound of a metal reducing substance and a culture medium component, produced in the culturing process of reducing bacteria.

2. The complex of claim 1, wherein the reducing bacteria are bacteria having at least one biomineralization function.

3. The complex of claim 1 or 2, wherein the culture medium component contains an electron donor and an electron acceptor.

4. The complex of any one of claims 1 to 3, wherein the reducing bacteria are iron-reducing bacteria.

5. The complex of claim 4, wherein the iron-reducing bacteria belong to the genus Shewanella.

6. The complex of any one of claims 1 to 5, containing phosphorus in a mass ratio of about a quarter of the whole.

7. The complex of any one of claims 1 to 6, wherein the complex is a fibrous material or substantially spherical aggregation of the fibrous material in sub-micron diameter.

8. The complex of any one of claims 1 to 7, wherein the complex has an adsorptive property and/or a reducing property.

9. A production method of the complex of any one of claims 1 to 8, comprising:
a process of culturing the reducing bacteria in a culture medium containing an oxide that can reduce the reducing bacteria;
a process in which a reduced product by the reducing bacteria and the culture medium component are bound to produce a bound product; and
a process in which the bound product is grown.

10. An adsorption method of adsorbing an ionic substance present in a solution by using the complex of any one of claims 1 to 8.

11. The adsorption method of claim 10, wherein the ionic substance is at least one metal selected from the group consisting of rare metal, precious metal, platinum-group metal, heavy metal and rare earth.

12. A reduction method of reducing at least one metal ion selected from the group consisting of a precious metal ion and a platinum-group metal ion by using the complex of any one of claims 1 to 8.

## Amended claims

### Amended claims under Art. 19.1 PCT

**1.** (Amended) A complex being a compound of a metal reducing substance and a culture medium component, produced in the culturing process of reducing bacteria, and containing phosphorus in a mass ratio of about a quarter of the whole.

**2.** The complex of claim 1, wherein the reducing bacteria are bacteria having at least one biomineralization function.

**3.** The complex of claim 1 or 2, wherein the culture medium component contains an electron donor and an electron acceptor.

**4.** The complex of any one of claims 1 to 3, wherein the reducing bacteria are iron-reducing bacteria.

**5.** The complex of claim 4, wherein the iron-reducing bacteria belong to the genus Shewanella.

**6.** (Deleted)

**7.** The complex of any one of claims 1 to 6, wherein the complex is a fibrous material or substantially spherical aggregation of the fibrous material in sub-micron diameter.

**8.** The complex of any one of claims 1 to 7, wherein the complex has an adsorptive property and/or a reducing property.

**9.** (Amended) A production method of the complex of any one of claims 1 to 8, comprising:
a process of culturing the reducing bacteria in a culture medium containing an oxide that can be reduced by the reducing bacteria;
a process in which a reduced product by the reducing bacteria and the culture medium component are bound to produce a bound product; and
a process in which the bound product is grown.

**10.** An adsorption method of adsorbing an ionic substance present in a solution by using the complex of any one of claims 1 to 8.

**11.** The adsorption method of claim 10, wherein the ionic substance is at least one metal selected from the group consisting of rare metal, precious metal, platinum-group metal, heavy metal and rare earth.

**12.** A reduction method of reducing at least one metal ion selected from the group consisting of a precious metal ion and a platinum-group metal ion by using the complex of any one of claims 1 to 8.
